(19) Europäisches Patentamt
European Patent Office
Office européen des brevets

(11) Publication number: 0 071 487
A2

(12) # EUROPEAN PATENT APPLICATION

(21) Application number: 82304045.6

(22) Date of filing: 30.07.82

(51) Int. Cl.³: C 07 D 307/86
C 07 C 49/755, C 07 C 49/743
C 07 C 45/68, C 07 C 45/71
C 07 C 45/67

(30) Priority: 31.07.81 US 289361

(43) Date of publication of application:
09.02.83 Bulletin 83/6

(84) Designated Contracting States:
BE CH DE FR GB IT LI NL

(71) Applicant: UNION CARBIDE CORPORATION
Old Ridgebury Road
Danbury Connecticut 06817(US)

(72) Inventor: Hunt, David Allen
5416 Highland Drive
Durham North Carolina 27712(US)

(74) Representative: Baverstock, Michael George Douglas et al,
BOULT, WADE & TENNANT 27 Furnival Street
London, EC4A 1PQ(GB)

(54) **Improved process for the preparation of carbofuran and novel intermediate.**

(57) A novel process is provided for the preparation of carbofuran. 1,2-Cyclohexanedione is alkylated with a beta-methallyl compound and the resulting product subjected to a Claisen rearrangement/aromatization to provide the carbofuran phenol. Reaction of the phenol with methyl isocyanate converts the phenol to the desired carbofuran.

The intermediate, 2-β-methallyloxy-2-cyclohexen-1-one, is considered to be novel.

EP 0 071 487 A2

IMPROVED PROCESS FOR THE PREPARATION OF CARBOFURAN AND NOVEL INTERMEDIATE

This invention relates, in general, to an improved process for the preparation of carbofuran. In one aspect this invention is directed to a simple, two step process for preparing carbofuran phenol from relatively inexpensive materials. In a further aspect, this invention involves the alkylation of 1,2-cyclohexanedione followed by either a catalytic or non-catalytic Claisen rearrangement/aromatization. The intermediate, 2-β-methallyloxy-2-cyclohexen-1-one, is also considered to be novel.

Carbofuran is a systemic insecticide acaricide, and nematicide currently manufactured and used in the United States and throughout the world. The product can be produced by the sequential conversion of o-nitrophenol to 2-β-methallyloxynitrobenzene, 2,3-dihydro-2,2-dimethyl-7-nitrobenzo-furan, 2,3-dihydro-2,2-dimethyl-7-benzofuranol, and finally 2,3-dihydro-2,2-dimethyl-7-benzofuranyl-N-methyl carbamate as described in U. S. - A - 3,320,286 which issued to FMC Corporation on May 16, 1967. However, the major disadvantages of the process are the expense and difficulty encountered in the preparation.

Most of the current procedures for the preparation of carbofuran as disclosed in the patent literature involve the alkylation of a 2-substituted phenol or phenol *per se* with β-methallyl chloride, followed by a Claisen rearrangement. The product, the 7-substituted-2,3-dihydro-2,2-dimethylbenzofuran, is then subjected to further transformations on the functional group at the 7-position in order to produce the desired benzofuranol. One process currently believed to be in use for the preparation of carbofuran is

described in U. S. - A - 3,320,286 and involves five distinct steps.

The process which involves the conversion of the aminobenzofuran to the benzofuranol via the diazonium salt is hazardous and requires special care in handling due to the potentially explosive nature of diazo salts. Thus, prior to the present invention, there was no simple process which utilized relatively inexpensive materials and avoided the hazards associated with diazonium salts.

Accordingly, one or more of the following objects can be achieved by the practice of this invention. It is an object of this invention to provide a process for the preparation of carbofuran. Another object of this invention is to provide a process which is simple and employs readily available and inexpensive starting materials. A further object is to provide a process which utilizes 1,2-cyclohexanedione as the starting material. Another object of the invention is to provide a simple two step process for the preparation of carbofuran phenol. These and other objects will readily become apparent to those skilled in the art in the light of the teachings hereinafter set forth.

The invention relates, in general, to an improved process for the preparation of carbofuran. The process comprises alkylating 1,2-cyclohexanedione with a beta-methallyl compound and subjecting the resulting product to a Claisen rearrangement/aromatization to provide carbofuran phenol. Reaction of the phenol with methyl isocyanate gives the carbofuran.

In the preferred route, 1,2-cyclohexanedione is O- and C- alkylated with β-methallyl chloride *via* base catalysis or O-alkylated with β-methallyl alcohol *via* acid catalysis to give the corresponding β-methallyldiosphenol III.

The process can be outlined as follows:

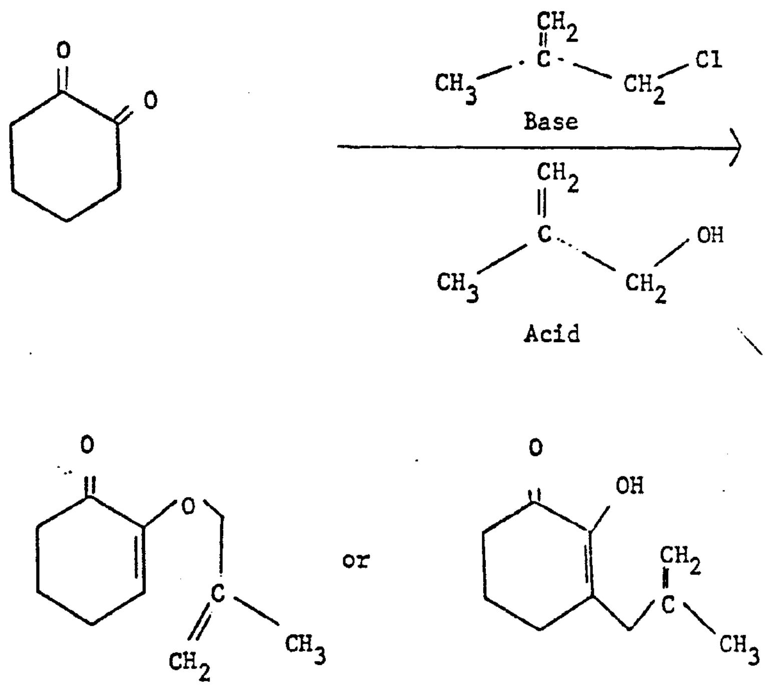


or

(Acid Catalyzed)

III

I

The key step in the process is the rearrangement of the disophenol III to the 3-substituted-1,2-dione I (shown as the enol) or the dihydrofuran derivative II:

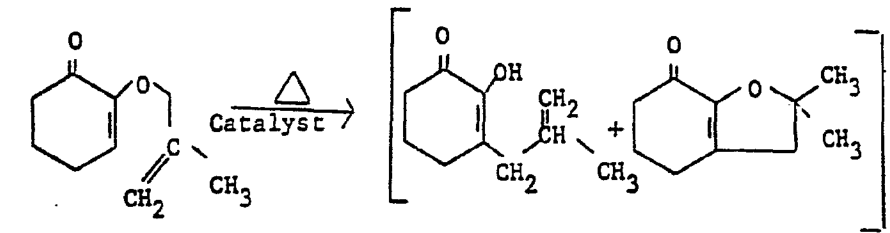


III I II

These intermediates are not isolated, but are further heated in the presence of an aromatization catalyst (such as palladium on various supports or sulfur) to yield the benzofuranol IV:

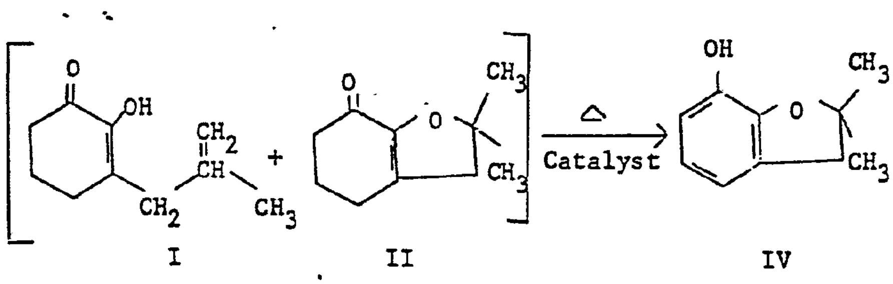


I II IV

The benzofuranol IV is then converted to carbofuran by standard carbamoylation procedures, such as by reaction with methyl isocyanate in the presence of a tertiary amine catalyst:

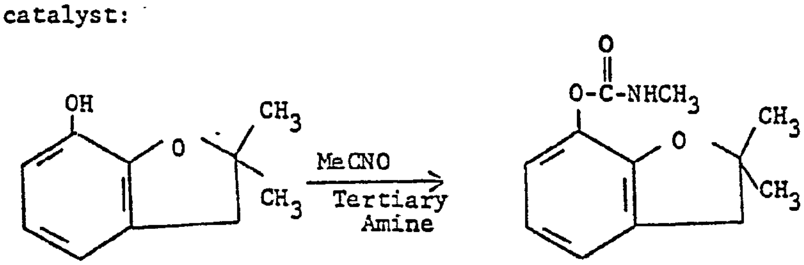


IV V

In practice, the process provides a simple, inexpensive route to carbofuran phenol and carbofuran itself. Moreover, the formation of hazardous intermediates is avoided and the method utilizes relatively inexpensive starting materials and simple chemistry.

As indicated above, the 2-beta-methallyloxy-2-cyclohexen-1-one (III), which is a novel compound, can undergo cyclization/aromatization to form the carbofuran phenol IV. Mechanistically, the 2-$\beta$-methallyloxy-2-cyclohexen-1-one can undergo the Claisen rearrangement to yield the diosphenol I (also formed via base catalyzed alkylation of 1,2-cyclohexanedione), aromatize, and cyclize onto oxygen or cyclize first to yield the dihydrofuran derivative II, followed by aromatization to yield the desired phenol.

As previously indicated, the first step in the process of this invention is the acid-catalyzed or base-catalyzed alkylation of cyclohexanedione at an oxygen or ring carbon atom. The technique is well known and disclosed in British - A - 1,258,554 which issued April 2, 1970 to Bayer A.G. and also reported by M. S. Gibson, J. Chem. Soc. (1962) 681. In the present invention beta-methallyl alcohol or a beta-methallyl halide, depending upon the catalyst used, is employed as the alkylating agent.

Reaction conditions and ratio of reactants are not necessarily critical and the process can be carried out under a variety of conditions.

A variety of acid and base alkylation catalysts can be used and the particular choice may depend upon economic reasons or other considerations. Catalysts which have been employed successfully in this invention include, among others, the acidic alkylation catalysts such as p-toluenesulfonic acid.

In the second step, the diophenol III undergoes the Claisen rearrangement and upon further heating in the presence of an aromatization catalyst, the intermediates formed undergoes aromatization to the benzofuranol. The Claisen rearrangement of enol or phenol allyl ethers is known. Additionally, the Claisen rearrangement of 2-allyl-oxy-2-cyclohexene-1-one to yield the 3-allyl-1,2-cyclohexanedione has been reported in the literature. However, the cyclization/aromatization of the novel compound 2-beta-methallyloxy-2-cyclohexene-1-one was not heretofore known.

It has been observed that in the absence of an aromatization catalyst, that is conducting the second step by heating alone, only small amounts of the benzofuranol IV is formed. Accordingly, to obtain sufficient yields of the desired phenol compound the use of a catalyst is preferred.

Although palladium and sulfur were used in the examples, any catalyst which is known to effect aromatization and which would otherwise not adversely affect the reactants or product can be used. Illustrative of other catalysts which can be employed, include, but are not limited to selenium, nickel, and platinum (and in general any noble metal catalyst and combinations thereof).

The temperatures employed in the second step are not necessarily critical and can vary over a wide range depending upon the reaction period. All that is necessary is that the desired rearrangement and aromatization be achieved.

In practice it has been found that temperatures of from 150°C to 350°C., and preferably from 150°C and 250°C were satisfactory to effect the desired reaction. Temperatures above and below this range can be employed but are less preferred.

The reaction time in which to effect rearrangement and aromatization can vary. However, reaction periods of up to between 1 and 8 hours have been found to be satisfactory.

As indicated, once the benzofuranol (carbofuran phenol) is obtained it can be converted to carbofuran by standard carbamoylation procedures known in the art. By reacting benzofuranol with methylisocyanate in the presence of a tertiary amine catalyst, excellent yields of the desired carbofuran are obtained.

The following Examples illustrate the best mode presently contemplated for the practice of this invention:

EXAMPLE 1

SYNTHESIS OF O- AND C-β-METHALLYL ALKYLATED 1,2-CYCLOHEXANEDIONE VIA BASE CATALYSIS

To a 500 ml 3-neck round bottom flask equipped with a magnetic stirrer, reflux condenser, $N_2$ inlet, and addition funnel were added, 1,2-cyclohexanedione (7.50g; 67.00mmol), acetone (250ml), potassium carbonate (27.62g; 200mmol) and potassium iodide (20.00g; 120 mmol). The reaction mixture was heated to reflux under $N_2$, and β-methallyl chloride (18.10g; 200 mmol) was added dropwise over a 15 minute period. The reaction mixture was refluxed with stirring under a $N_2$ blanket for 17 hours. The mixture was then allowed to cool to room temperature and was filtered in order to remove salts. The filtrate was concentrated (rotary evaporation) to yield 18.75g of a light brown oil which was distilled in vacuo to give 6.79g (61 percent) of the desired product as a pale yellow oil, bp 93-98°C/5-6 torr. $^1H$ nmr analysis revealed a mixture of both O- and C-alkylated product and their corresponding enol tautomers. $^1H$ nmr (relative intensities with respect to the 4.21 absorption are reported) ($CDCl_3$): $\delta$1.35-2.82 (m, 27.6, aliphatic- $CH_2$-$CH_3$), 3.02 (s, 3.2,-O-$CH_2$), 4.21 (s, 1, -OH), 4.83 (m, 4.9, vinylic- $CH_2$), 5.88 (t,1, endocyclic definic-CH), 6.10 (t, 1.2, endocyclic olefinic-CH), 6.28 (bds, 1.6, exocyclic vinylic $CH_2$). IR (Film): 910, 1140, 1200, 1225, 1390, 1665, 3440 $cm.^{-1}$

## EXAMPLE 2

### SYNTHESIS OF O-β-METHALLYL ALKYLATED 1,2-CYCLOHEXANEDIONE VIA ACID CATALYSIS

To a 500 ml round bottom flask equipped with an 18 inch Oldershaw column (10 trays), Dean-Stark trap, reflux condenser, heating mantle, and magnetic stirrer were added 1,2-cyclohexanedione (100.00 g; 893 mmol), benzene (125 ml), β-methallyl alcohol (160 ml), and p-toluenesulfonic acid (0.5 g). The mixture was then rigorously refluxed for 24 hours and water was collected. The mixture was permitted to cool, and 0.6 g $CaCO_3$ was added. The mixture was filtered and concentrated (rotary evaporation) to yield 146.27 g of a brown oil which was distilled *in vacuo* to give 114.86 g (77 percent) of the desired product as a pale yellow oil, bp 122-129.5°C/10-10.5 torr. $^1$Hnmr ($CDCl_3$): δ 0.89-2.67 (m,9, aliphatic-$CH_2$-$CH_3$), 3.00 (s,2,-$OCH_2$), 4.61 (m, 2, vinylic-$CH_2$), 5.94 (m,1, endocyclic vinyl-CH). IR (film): 1380, 1665, 3420 $cm.^{-1}$

## EXAMPLE 3

### NON-CATALYTIC CLAISEN REARRANGEMENT/ AROMATIZATION OF 2-β-METHALLYLOXY-2-CYCLOHEXENE-1-ONE

To a 50 ml single neck round bottom flask equipped with a magnetic stirrer and $N_2$ inlet were added 2-β-methallyloxy-2-cyclohexene-1-one (2.50 g; 15.54 mmol) and anhydrous $MgCl_2$ (0.2 g). The mixture was heated with stirring under a $N_2$ blanket at 184°C for 6.5 hours. The mixture was allowed to cool and was distilled *in vacuo* to yield three fractions: i) 2,2-dimethyl-2,3-dihydrobenzofuran, 0.55 g

23.91 percent yield), bp 49-52°C/3.0 torr; 'H nmr ($CDCl_3$): δ 1.44 (s,6,gem-$CH_3$); 2.96 (s,2,benzylic-C$\underline{H}_2$; 6.80-7.23 (m,4,Ar$\underline{H}$); b) an unidentified fraction containing mainly aliphatic material based on 'H nmr analysis, 0.22 g, bp 76-90°C/3.0 torr; c) carbofuran phenol, 0.50g (19.38 percent), bp 92-97°C/3.0 torr; 'H nmr ($CDCl_3$):δ 1.48 (2,6, gem -$CH_3$); 2.98 (s,2, benzylic -C$\underline{H}_2$); 5.38 (bds, 1,-O$\underline{H}$); 6.66 (s,3,Ar$\underline{H}$).

## EXAMPLE 4

### CATALYTIC CLAISEN REARRANGEMENT/ AROMATIZATION OF 2- -METHALLYLOXY- 2-CYCLOHEXENE-1-ONE

2-β-Methallyloxy-2-cyclohexene-1-one (3.00 g; 18.1 mmol) and 60 mg of 0.3 percent Pd/alumina catalyst were placed into a 10 ml round bottom flask equipped with a magnetic stirrer, oil bath, and $N_2$ inlet. The mixture was heated at 150°C for 6 hours under a $N_2$ blanket, at which point sulfur powder (0.53 g; 18.1 mmol) was added, and the mixture was heated an additional 5 hours at 185-203°C under a $N_2$ blanket. The mixture was allowed to cool and 'H nmr spectrum of the mixture was recorded. NMR analysis revealed virtually quantitative conversion to carbofuran phenol.

## EXAMPLE 5

The carbofuran phenol from Examples 3 and 4 is then reacted with methyl isocyanate in the presence of a tertiary amine catalyst to give carbofuran (2,3-dihydro-2,2-dimethyl-7-benzofuranyl-N-methyl carbamate).

CLAIMS:

1. A process for the preparation of carbofuran which comprises the steps of:

(a) reacting 1,2-cyclohexanedione with a beta-methallyl alcohol or halide in the presence of an alkylation catalyst to form 2-beta-methallyloxy-2-cyclohexen-1-one;

(b) heating the 2-beta-methallyloxy-2-cyclohexen-1-one to a temperature sufficient to cause rearrangement and aromatization to benzofuranol; and

(c) reacting the benzofuranol with methyl isocyanate to form carbofuran.

2. A process for the preparation of benzofuranol which comprises the steps of:

(a) reacting 1,2-cyclohexanedione with a beta-methallyl alcohol or halide in the presence of an alkylation catalyst to form 2-beta-methallyloxy-2-cyclohexen-1-one; and

(b) heating the 2-beta-methallyloxy-2-cyclohexen-1-one to a temperature sufficient to cause rearrangement and aromatization to benzofuranol.

3. A process as claimed in claim 1 or claim 2

wherein the alkylation catalyst is an acidic catalyst.

4. A process as claimed in claim 3 wherein the acidic catalyst is p-toluenesulfonic acid.

5. A process as claimed in claim 1 or claim 2 wherein the alkylation catalyst is a basic catalyst.

6. A process as claimed in claim 5 wherein the basic catalyst is potassium carbonate.

7. A process as claimed in any one of the preceding claims wherein the beta-methallyl halide is beta-methallyl chloride.

8. A process as claimed in any one of the preceding claims wherein step (b) is carried out in the presence of an aromatization catalyst.

9. A process as claimed in claim 8 wherein the aromatization catalyst is palladium.

10. A process as claimed in claim 8 wherein the aromatization catalyst is sulfur.

11. A process for the preparation of 2-beta-methallyloxy-2-cyclohexen-1-one which comprises reacting 1,2-cyclohexanedione with beta-methallyl alcohol in the presence of an acidic alkylating catalyst.

12. 2-Beta-methallyloxy-2-cyclohexen-1-one.

MGB/SJW/DR/EA656